# FASCICULE DE BREVET EUROPEEN

(11) **EP 3 057 877 B1**
(45) Date de publication et mention de la délivrance du brevet: **06.12.2017**
(21) Numéro de dépôt: 14799509.6
(22) Date de dépôt: 13.10.2014
(51) Int. Cl.: B65D 55/10, B65D 25/10, B05B 11/00, A61M 11/06, A61M 15/00

(54) **DISPOSITIF DE CONDITIONNEMENT D'UN DISTRIBUTEUR DE PRODUIT FLUIDE**
VERFAHREN UND VORRICHTUNG ZUR VERPACKUNG EINES FLÜSSIGPRODUKTSPENDERS
DEVICE FOR PACKAGING A FLUID PRODUCT DISPENSER

(30) Priorité: 15.10.2013 FR 1360012
(43) Date de publication de la demande: 24.08.2016
(73) Titulaire: Aptar France SAS, 27110 Le Neubourg (FR)
(72) Inventeur: LE MANER, François, 27400 La Vallee Montaure (FR)
(74) Mandataire: CAPRI
(86) Numéro de dépôt international: PCT/FR2014/052599
(87) Numéro de publication internationale: WO 2015/055931

(56) Documents cités:
- JP-A- 2004 208 973
- JP-A- 2011 093 584
- US-A- 2 084 540
- US-A- 3 376 866
- US-B1- 6 708 846

## Description

La présente invention concerne un dispositif de conditionnement d'un distributeur de produit fluide.

Les distributeurs de produit fluide sont bien connus, notamment pour la distribution de produits fluides pharmaceutiques, cosmétiques ou du domaine de la parfumerie. Ces dispositifs comportent généralement au moins un réservoir contenant du produit fluide et un ensemble de distribution à actionnement manuel pour distribuer ledit produit fluide. Un problème qui se pose avec ces dispositifs concerne leur sécurité en période de stockage, lorsqu'ils ne sont pas utilisés. Ainsi, par exemple, une chute du dispositif peut engendrer un actionnement partiel ou total non souhaité de l'ensemble de distribution. Ceci peut aussi se produire en fin d'assemblage et/ou de remplissage du dispositif. Un tel actionnement total ou partiel non souhaité peut poser des problèmes importants, par exemple de perte de dose, de perte d'étanchéité ou de dysfonctionnement ultérieur du dispositif. Ces problèmes sont particulièrement néfastes pour les dispositifs de distribution du type unidose ou bidose, destinés à distribuer seulement une ou deux doses de produit fluide, ainsi que pour les dispositifs du type autoinjecteur.

Les documents US3376866, JP2004208973, JP2011093584 et US2084540 décrivent des dispositifs de l'art antérieur.

La présente invention a pour objectif de fournir un dispositif de conditionnement d'un distributeur de produit fluide pour limiter ou éviter les inconvénients précités.

La présente invention a notamment pour but de fournir un tel dispositif qui évite tout risque d'actionnement intempestif du distributeur en cas de chute du dispositif de conditionnement contenant ledit distributeur.

La présente invention a donc pour objet un ensemble comportant un dispositif de conditionnement d'un distributeur de produit fluide et ledit distributeur, ledit distributeur comportant au moins un réservoir contenant du produit fluide et un ensemble de distribution à actionnement manuel pour distribuer ledit produit fluide, ledit dispositif de conditionnement comportant un boitier pourvu d'un corps de base et d'un couvercle amovible dudit corps de base à partir d'une position fermée, dans laquelle ledit distributeur est conditionné dans ledit boitier, vers une position ouverte, dans laquelle ledit distributeur peut être retiré dudit boitier, ledit boitier comportant au moins une projection saillante adaptée à coopérer avec ledit distributeur au moins en position fermée du couvercle pour bloquer ledit ensemble de distribution et empêcher son actionnement, ledit distributeur comportant une partie de corps pourvue d'au moins une ouverture de blocage recevant ladite projection saillante de telle sorte que lorsque ladite projection saillante est disposée dans ladite ouverture de blocage, ladite projection saillante bloque l'actionnement dudit ensemble de distribution.

Avantageusement, au moins une de ladite au moins une projection saillante est disposée dans la face interne dudit corps de base.

Avantageusement, au moins une de ladite au moins une projection saillante est disposée dans la face interne dudit couvercle.

Avantageusement, ledit distributeur comprend une tête de distribution pourvue d'un orifice de distribution du produit fluide, ledit ensemble de distribution étant une pompe ou une valve doseuse actionnée par déplacement de ladite tête de distribution par rapport au réservoir, ladite au moins une projection saillante, en position fermée du couvercle, empêchant ledit déplacement de ladite tête de distribution.

Avantageusement, ladite tête de distribution comporte ladite ouverture de blocage.

Avantageusement, ledit ensemble de distribution comprend un piston déplaçable dans un réservoir pour expulser le produit fluide hors dudit réservoir, et une tige de piston connectée audit piston lors de l'actionnement, ledit piston étant actionnée par déplacement de ladite tige de piston par rapport audit réservoir, ladite au moins une projection saillante, en position fermée du couvercle, empêchant ledit déplacement de ladite tige de piston par rapport audit réservoir.

Avantageusement, ledit distributeur est un autoinjecteur.

Avantageusement, ledit boitier comporte en outre des moyens de positionnement du distributeur.

Avantageusement, lesdits moyens de positionnement comprennent ladite au moins une projection saillante.

Avantageusement, ledit couvercle est mobile, notamment pivotant, par rapport audit corps de base, de sorte qu'en position ouverte dudit couvercle, ledit distributeur peut être installé et retiré dudit boitier.

En variante, ledit couvercle est fixé de manière arrachable audit corps de base.

Ces caractéristiques et avantages et d'autres de la présente invention apparaîtront plus clairement au cours de la description détaillée suivante, faite en référence aux dessins joints, donnés à titre d'exemples non-limitatifs, et sur lesquels :
la figure 1 est une vue schématique en perspective du dispositif de conditionnement selon un mode de réalisation avantageux, en position ouverte, avec le distributeur en cours d'installation dans le dispositif de conditionnement,
la figure 2 est une vue schématique en perspective découpée selon un plan vertical du dispositif de la figure 1 en position fermée,
la figure 3 est une vue de détail en section transversale verticale,
les figure 4 à 6 sont des vues schématiques de côté de trois variantes d'un autre mode de réalisation avantageux de l'invention, avec un boitier sous forme de blister,
la figure 7 est une vue schématique en section transversale du blister de la figure 4 en position fermée recevant un distributeur du type bidose, et
la figure 8 est une vue similaire à celle de la figure 7 du blister de la figure 6 en position fermée recevant un distributeur du type bidose.

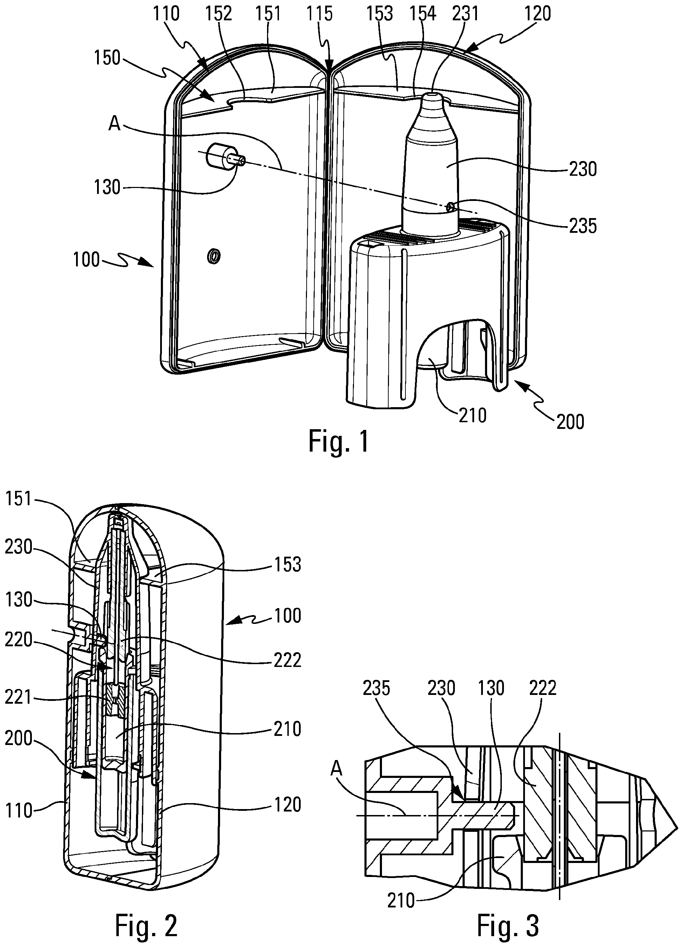

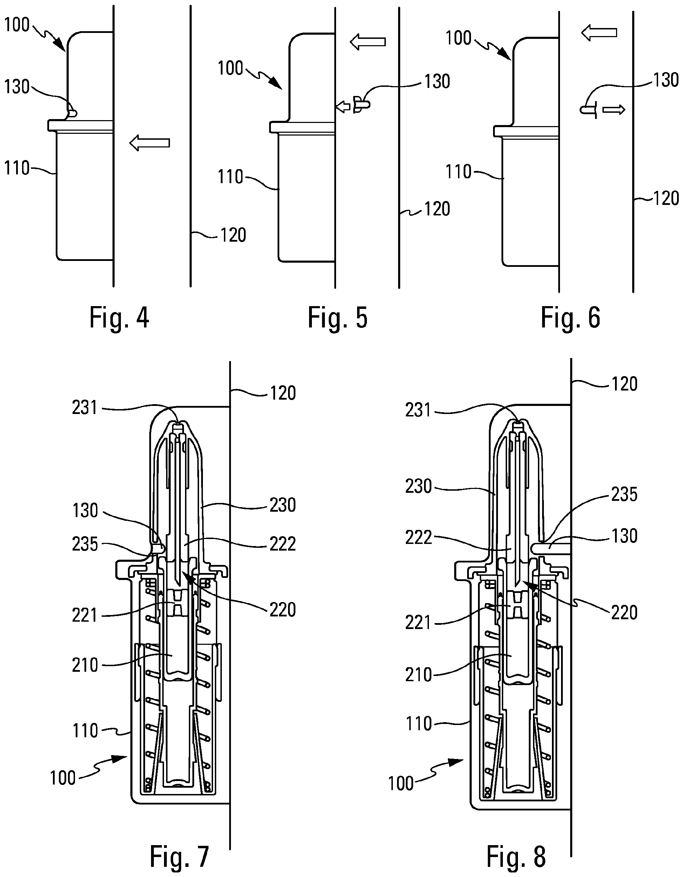

Dans la présente description, le terme "produit fluide" comprend les produits liquides, pâteux ou pulvérulents, et les termes "supérieur", "inférieur", "vertical" et "horizontal" se réfèrent à la position droite du dispositif représenté sur les figures 1 et 4.

Les figures 1 à 3 représentent un premier mode de réalisation avantageux de la présente invention, comportant un boitier 100 formant partie d'un dispositif de conditionnement. Sur cette figure 1, le boitier 100 est en position ouverte, avec un distributeur 200 en cours d'installation.

Le boitier 100 comporte un corps de base 110 et un couvercle 120 mobile, notamment pivotant, par rapport audit corps de base 110 entre une position ouverte, dans laquelle ledit distributeur 200 peut être installé et retiré dudit boitier 100, et une position fermée, dans laquelle ledit distributeur 200 est conditionné dans ledit boitier 100. Comme représenté sur la figure 1, le couvercle 120 peut pivoter autour d'un bord latéral 115 du corps de base 110 formant charnière. Le boitier peut être réalisé en une seule pièce, avec une charnière film formant le bord latéral de pivotement, ou en deux pièces, avec une charnière mécanique entre le corps de base et le couvercle.

Le distributeur 200 peut être de type quelconque, par exemple un dispositif multidoses, c'est-à-dire un dispositif comportant une pompe ou une valve en tant qu'ensemble de distribution, montée sur un réservoir contenant une pluralité de doses. Le distributeur peut aussi être un dispositif d'injection, tel qu'un autoinjecteur. Dans l'exemple représenté sur les figures, le distributeur 200 est un unidose (ou bidose), c'est-à-dire qu'il ne contient qu'une seule (ou seulement deux) dose(s) de produit fluide à distribuer. Bien entendu, cet exemple n'est pas limitatif.

Dans l'exemple représenté, le distributeur 200 comporte un réservoir 210 contenant une ou deux dose(s) de produit fluide à distribuer. Le distributeur 200 comporte en outre une partie de corps 230 pourvue d'au moins une ouverture de blocage 235. Cette partie de corps 230 est avantageusement une tête de distribution, comme visible sur les figures 1 et 2, pourvue d'un orifice de distribution 231 du produit fluide.

L'ensemble de distribution 220 peut être une pompe ou une valve doseuse actionnée par déplacement de ladite tête de distribution 230 par rapport au réservoir 210. En variante, comme visible sur la figure 2, ledit ensemble de distribution 220 peut comprendre un piston 221 déplaçable dans le réservoir 210 pour expulser le produit fluide hors dudit réservoir, et une tige de piston 222 connectée audit piston 221 lors de l'actionnement, ledit piston 221 étant actionnée par déplacement de ladite tige de piston 222 par rapport audit réservoir 210.

Selon l'invention, ledit boitier 100 comporte au moins une projection saillante 130 adaptée à coopérer avec ledit distributeur 200 au moins en position fermée du couvercle 120 pour bloquer ledit ensemble de distribution 220 et empêcher son actionnement.

Avantageusement, comme visible sur la figure 1, ladite au moins une projection saillante 130 est disposée dans la face interne dudit corps de base 110. En variante, ladite au moins une projection saillante 130 peut aussi être disposée dans la face interne dudit couvercle 120. Si souhaitable, on peut prévoir deux projections saillantes 130, une dans le corps de base 110 et l'autre dans le couvercle 120, auquel cas le distributeur 200 comporte également deux ouvertures de blocage 235.

Dans l'exemple de la figure 1, le distributeur 200 est mis en place dans le boitier 100, avec une ouverture de blocage 235 du distributeur venant s'emmancher sur une projection saillante 130 prévue dans le corps de base 110, dans le sens de l'axe A représenté sur la figure 1.

Les figures 4 à 8 illustrent un autre mode de réalisation, dans lequel le boitier 100 forme un blister pour le distributeur 200, avec le couvercle 120 fixé de manière arrachable audit corps de base 110. Le corps de base peut être réalisé sous la forme d'une coque thermoformée. La projection saillante 130 peut être formée monobloc dans le corps de base 110, comme visible sur les figures 4 et 7, ou sur le couvercle 120, comme visible sur les figures 6 et 8. Dans ces deux variantes, la projection saillante 130 peut soit être formée directement dans le corps de base ou le couvercle lors de leur fabrication, soit être formée séparément puis collée ou clipsée sur le corps de base 110, comme visible sur la figure 5, ou sur le couvercle 120, comme visible sur la figure 6.

Comme visible sur les figures 2, 3, 7 et 8, en position de blocage, la projection 130 s'étend à l'intérieur de la tête de distribution 230 au-dessus du bord supérieur du réservoir 210, empêchant de ce fait tout déplacement relatif entre le réservoir 210 et la tête de distribution 230.

Ainsi, en position de blocage, aucun actionnement, même partiel, n'est possible tant que la projection saillante 130 est en position de blocage, y compris pendant une chute du dispositif de conditionnement.

Avantageusement, ledit boitier 100 peut en outre comporter des moyens de positionnement 150 du distributeur 200, notamment pour éviter la chute du distributeur lors de l'ouverture du boitier. Ces moyens de positionnement 150 peuvent notamment comprendre ladite au moins une projection saillante 130. Dans l'exemple des figures 1 à 3, ces moyens de positionnement peuvent aussi comporter une bride de positionnement 151 formée dans le corps de base 110 et une bride correspondante 153 dans le couvercle 120. Chaque bride 151, 153 comporte une découpe centrale 152, 154, permettant le positionnement de ladite tête de distribution 230. Dans l'exemple des figures 4 à 8, ces moyens de positionnement peuvent comporter un corps de base 110 dont la forme est adaptée à la forme du distributeur 200, ledit distributeur 200 étant alors coincé dans ledit corps de base même après que le couvercle 120 a été arraché dudit corps de base.

Bien que la présente invention ait été décrite en référence à plusieurs modes de réalisation particuliers, il est entendu que la présente invention n'est pas limitée par ceux-ci mais qu'au contraire l'homme du métier peut y apporter toutes modifications utiles sans sortir du cadre de la présente invention tel que défini par les revendications annexées.

## Revendications

1. Ensemble comportant un dispositif de conditionnement d'un distributeur (200) de produit fluide et ledit distributeur, ledit distributeur comportant au moins un réservoir (210) contenant du produit fluide et un ensemble de distribution (220) à actionnement manuel pour distribuer ledit produit fluide, ledit dispositif de conditionnement comportant un boitier (100) pourvu d'un corps de base (110) et d'un couvercle (120) amovible dudit corps de base (110) à partir d'une position fermée, dans laquelle ledit distributeur (200) est conditionné dans ledit boitier (100), vers une position ouverte, dans laquelle ledit distributeur (200) peut être retiré dudit boitier (100), ledit boitier (100) comportant au moins une projection saillante (130) adaptée à coopérer avec ledit distributeur (200) au moins en position fermée du couvercle (120) pour bloquer ledit ensemble de distribution (220) et empêcher son actionnement, **caractérisé en ce que** ledit distributeur (200) comporte une partie de corps (230) pourvue d'au moins une ouverture de blocage (235) recevant ladite projection saillante (130) de telle sorte que lorsque ladite projection saillante (130) est disposée dans ladite ouverture de blocage (235), ladite projection saillante (130) bloque l'actionnement dudit ensemble de distribution (220), au moins une de ladite au moins une projection saillante (130) étant disposée dans la face interne dudit couvercle (120).

2. Ensemble selon la revendication 1, dans lequel au moins une de ladite au moins une projection saillante (130) est disposée dans la face interne dudit corps de base (110).

3. Ensemble selon l'une quelconque des revendications précédentes, dans lequel ledit distributeur (200) comprend une tête de distribution (230) pourvue d'un orifice de distribution (231) du produit fluide, ledit ensemble de distribution (220) étant une pompe ou une valve doseuse actionnée par déplacement de ladite tête de distribution (230) par rapport au réservoir (210), ladite au moins une projection saillante (130), en position fermée du couvercle (120), empêchant ledit déplacement de ladite tête de distribution (230).

4. Ensemble selon l'une quelconque des revendications précédentes, dans lequel ladite tête de distribution (230) comporte ladite ouverture de blocage (235).

5. Ensemble selon la revendication 1 ou 2, dans lequel ledit ensemble de distribution (220) comprend un piston (221) déplaçable dans un réservoir (210) pour expulser le produit fluide hors dudit réservoir, et une tige de piston (222) connectée audit piston (221) lors de l'actionnement, ledit piston (221) étant actionnée par déplacement de ladite tige de piston (222) par rapport audit réservoir (210), ladite au moins une projection saillante (130), en position fermée du couvercle (120), empêchant ledit déplacement de ladite tige de piston (222) par rapport audit réservoir (210).

6. Ensemble selon la revendication 5, dans lequel ledit distributeur est un autoinjecteur.

7. Ensemble selon l'une quelconque des revendications précédentes, dans lequel ledit boitier (100) comporte en outre des moyens de positionnement (150) du distributeur (200).

8. Ensemble selon la revendication 7, dans lequel lesdits moyens de positionnement (150) comprennent ladite au moins une projection saillante (130).

9. Ensemble selon l'une quelconque des revendications précédentes, dans lequel ledit couvercle (120) est mobile, notamment pivotant, par rapport audit corps de base (110), de sorte qu'en position ouverte dudit couvercle (120), ledit distributeur (200) peut être installé et retiré dudit boitier (100).

10. Ensemble selon l'une quelconque des revendications 1 à 9, dans lequel ledit couvercle (120) est fixé de manière arrachable audit corps de base (110).

## Patentansprüche

1. Anordnung mit einer Vorrichtung zur Verpackung eines Flüssigproduktspenders (200) sowie dem genannten Flüssigproduktspender, wobei der Spender zumindest einen Vorratsbehälter (210) umfasst, der ein Flüssigprodukt enthält, und eine manuell betätigbare Abgabevorrichtung (220) zur Abgabe des Flüssigprodukts, wobei die Verpackungsvorrichtung ein Gehäuse (100) umfasst, welches aus einem Grundkörper (110) und einem vom Grundkörper (110) aus einer geschlossenen Stellung heraus abnehmbaren Deckel (120) umfasst, wobei der genannte Spender (200) in dem Gehäuse (100) in einer geöffneten Stellung verpackt ist, in der der Spender (200) aus dem Gehäuse (100) entfernbar ist, wobei das Gehäuse (100) zumindest einen Vorsprung (130) umfasst, welcher zum Zusammenwirken mit dem Spender (200) zumindest bei geschlossener Stellung des Deckels (120) ausgelegt ist, um die Abgabevorrichtung (220) zu blockieren und deren Betätigung zu verhindern, **dadurch gekennzeichnet, dass** der Spender (200) ein Gehäuseteil (230) umfasst, das mit zumindest einer Arretieröffnung (235) versehen ist, welche den Vorsprung (130) derart aufnimmt, dass besagter Vorsprung (130) in der Arretieröffnung (235) angeordnet ist, wobei der Vorsprung (130) die Betätigung besagter Abgabevorrichtung (220) blockiert, wobei zumindest einer des zumindest einen Vorsprungs (130) an der Innenseite des Deckels (120) angeordnet ist.

2. Anordnung nach Anspruch 1, bei der zumindest einer des zumindest einen Vorsprungs (130) an der Innenseite des Grundkörpers (110) angeordnet ist.

3. Anordnung nach einem der voranstehenden Ansprüche, bei der der Spender (200) einen Abgabekopf (230) umfasst, der mit einer Abgabeöffnung (231) für das Flüssigprodukt versehen ist, wobei die Abgabevorrichtung (220) eine Pumpe oder ein Dosierventil ist, welche bzw. welches durch Verschieben des Abgabekopfs (230) bezüglich des Vorratsbehälters (210) betätigt wird, wobei der mindestens eine Vorsprung (130) bei geschlossener Stellung des Deckels (120) das Verschieben des Abgabekopfes (230) verhindert.

4. Anordnung nach einem der voranstehenden Ansprüche, bei der der Abgabekopf (230) die Arretieröffnung (235) umfasst.

5. Anordnung nach Anspruch 1 oder 2, bei der die Abgabevorrichtung (220) einen Kolben (221) umfasst, der in einem Vorratsbehälter (210) verschiebbar ist, um das Flüssigprodukt aus dem Vorratsbehälter herauszudrücken, sowie eine mit dem Kolben (221) zu dessen Betätigung verbundene Kolbenstange (222), wobei der Kolben (221) durch Verschieben der Kolbenstange (222) bezüglich des Vorratsbehälters (210) betätigt wird, wobei der zumindest eine Vorsprung (130) bei geschlossener Stellung des Deckels (120) das Verschieben der Kolbenstange (222) bezüglich des Vorratsbehälters (210) verhindert.

6. Anordnung nach Anspruch 5, bei der der Spender ein Autoinjektionsgerät ist.

7. Anordnung nach einem der voranstehenden Ansprüche, bei der das Gehäuse (100) darüber hinaus Positioniermittel (150) für den Spender (200) umfasst.

8. Anordnung nach Anspruch 7, bei der die Positioniermittel (150) den zumindest einen Vorsprung (130) umfassen.

9. Anordnung nach einem der voranstehenden Ansprüche, bei der der Deckel (120) bezüglich des Grundkörpers (110) beweglich, insbesondere schwenkbar, angeordnet ist, derart, dass bei geöffneter Stellung des Deckels (120) der Spender (200) in das Gehäuse (100) eingeführt bzw. aus diesem entnommen werden kann.

10. Anordnung nach einem der voranstehenden Ansprüche 1 bis 9, bei der der Deckel (120) am Grundkörper (110) abreißbar befestigt ist.

## Claims

1. An assembly comprising a packaging device for packaging a fluid dispenser (200) together with said dispenser, said dispenser comprising at least one reservoir (210) containing fluid, and a dispenser unit (220) that is actuated manually so as to dispense said fluid, said packaging device comprising a casing (100) that is provided with a base body (110) and with a cover (120) that is separable from said base body (110), starting from a closed position in which said dispenser (200) is packaged in said casing (100) and going to an open position in which said dispenser (200) may be removed from said casing (100), said casing (100) including at least one projection (130) that is adapted to co-operate with said dispenser (200) at least in the closed position of the cover (120), so as to block said dispenser unit (220) and prevent it from being actuated, the assembly being **characterized in that** said dispenser (200) includes a body portion (230) that is provided with at least one blocking opening (235) that receives said projection (130), such that when said projection (130) is arranged in said blocking opening (235), said projection (130) prevents said dispenser unit (220) from being actuated, at least one of said at least one projection (130) being arranged on the inside face of said cover (120).

2. An assembly according to claim 1, wherein at least one of said at least one projection (130) is arranged on the inside face of said base body (110).

3. An assembly according to any preceding claim, wherein said dispenser (200) includes a dispenser head (230) that is provided with a dispenser orifice (231) for dispensing the fluid, said dispenser unit (220) being a pump or a metering valve that is actuated by moving said dispenser head (230) relative to the reservoir (210), said at least one projection (130), in the closed position of the cover (120), preventing said movement of said dispenser head (230).

4. An assembly according to any preceding claim, wherein said dispenser head (230) includes said blocking opening (235).

5. An assembly according to claim 1 or claim 2, wherein said dispenser unit (220) includes a piston (221) that is movable in a reservoir (210) so as to expel the fluid out from said reservoir, and a piston rod (222) that is connected to said piston (221) during actuation, said piston (221) being actuated by moving said piston rod (222) relative to said reservoir (210), said at least one projection (130), in the closed position of the cover (120), preventing said movement of said piston rod (222) relative to said reservoir (210).

6. An assembly according to claim 5, wherein said dispenser is an autoinjector.

7. An assembly according to any preceding claim, wherein said casing (100) further includes positioner means (150) for positioning the dispenser (200).

8. An assembly according to claim 7, wherein said positioner means (150) comprise said at least one projection (130).

9. An assembly according to any preceding claim, wherein said cover (120) is movable, in particular pivotable, relative to said base body (110), such that in the open position of said cover (120), said dispenser (200) may be installed in and removed from said casing (100).

10. An assembly according to any one of claims 1 to 9, wherein said cover (120) is fastened in tear-off manner to said base body (110).
